# EUROPEAN PATENT SPECIFICATION

(11) **EP 2 586 464 B1**
(45) Date of publication and mention of the grant of the patent: **25.07.2018**
(21) Application number: 11797572.2
(22) Date of filing: 02.06.2011
(51) Int. Cl.: A61K 9/14, A61K 9/16, A61K 31/55, A61K 9/20, A61P 7/10

(54) **TOLVAPTAN SOLID DISPERSION AND ITS PREPARATION METHOD**
FESTE TOLVAPTANDISPERSION UND DEREN HERSTELLUNGSVERFAHREN
DISPERSION SOLIDE DE TOLVAPTAN ET SON PROCÉDÉ DE PRÉPARATION

(30) Priority: 25.06.2010 CN 201010224884
(43) Date of publication of application: 01.05.2013
(73) Proprietor: Jiangsu Hengrui Medicine Co. Ltd., Jiangsu 222047 (CN)
(72) Inventor: WU, Yuxia, Lianyungang Jiangsu 222047 (CN); MAO, Shujun, Lianyungang Jiangsu 222047 (CN); CHEN, Hao, Lianyungang Jiangsu 222047 (CN)
(74) Representative: Regimbeau
(86) International application number: PCT/CN2011/075131
(87) International publication number: WO 2011/160541

(56) References cited:
- WO-A2-2008/156217
- WO-A2-2008/156217
- JP-A- H1 121 241
- JP-A- 11 021 241
- "Crospovidone ED - Rowe Raymond C; Sheskey Paul J; Weller Paul J", 26 November 2002 (2002-11-26), HANDBOOK OF PHARMACEUTICAL EXCIPIENTS, PHARMACEUTICAL PRESS, LONDON, UK, PAGE(S) 184, XP002719370, ISBN: 978-0-85369-537-0 * page 184, paragraph 7 *

## Description

### FIELD OF THE INVENTION

The present invention belongs to the field of chemical pharmacy. It discloses a tolvaptan solid dispersion comprising cross-linked polyvinylpyrrolidone.

### BACKGROUND OF THE INVENTION

Tolvaptan,7-chloro-5-hydroxy-1-[2-methyl-4-(2-methylbenzoylamino)benzoyl]-2,3,4, 5,-tetrohydro-1H-benzoazepine represented by the following general formula is used as a vasopressin antagonist.

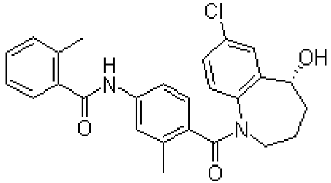

The U.S. Food and Drug Administration has approved tolvaptan (vasopressin receptor 2 antagonist)manufactured by Otsuka Pharmaceutical Co. to be marketed. In heart failure model, tolvaptan only exhibit hydragogue diuresis effect, decrease heart preload significantly, while has no effect on heart afterload and renal failure. Many clinical trials of treating heart failure and hyponatremia has proved that tolvaptan can decrease fluid retention and increase the serum sodium concentration. Tolvaptan can also release the symptom of pulmonary congestion, lose weight, protect the kidney function and has no severe adverse effect. It has been proved that tolvaptan is specifically useful in the treatment of clinical significant hypervolemic and normovolemic hyponatremia associated with congestive heart failure, cirrhosis and the syndrome of inappropriate antidiuretic hormone(SIADH).

Tolvaptan, white crystal or crystalline powder, is very slightly soluble in the water. Therefore it is inevitably for tolvaptan to have a low bioavailability when administered orally.

In order to solve this problem, JP11021241A disclosed a preparation method of tolvaptan solid preparation composition comprising the following steps: tolvaptan and hydroxypropylcellulose are dissolved in the organic solvent at a certain ratio, the organic solvent is removed via spray dry method to obtain tolvaptan solid dispersion composition, other pharmaceutically acceptable excipients are added to obtain the final solid preparation. WO 2008/156217 discloses an amorphous composite comprised of tolvaptan and hydroxypropylcellulose, which is incorporated into a solid preparation.

### DESCRIPTION OF THE INVENTION

The inventors unexpectedly found that the tolvaptan solid dispersion obtained by mixing tolvaptan and the carrier comprising cross-linked polyvinylpyrrolidone showed superior solubility and stability of the active ingredient than the preparation containing hydroxypropylcellulose in the art. Moreover, it is more convenient to be prepared. The inventors also found that adding other water soluble polymers to the present formulation can further improve the dissolution rate.

An object of the present invention is to provide a novel tolvaptan solid dispersion which exhibits improved solubility and bioavailability than the traditional preparation, and the preparation method of the same.

The other object of the present invention is to provide a pharmaceutical composition comprising tolvaptan solid dispersion which is administered orally.

In one embodiment of the present invention, there is provided a solid dispersion comprising amorphous tolvaptan or the salt thereof as the active ingredient and cross-linked polyvinylpyrrolidone. The ratio of tolvaptan or the salt thereof and cross-linked polyvinylpyrrolidone by weight is in the range of 1:0.1~10, preferably 2:1. The present solid dispersion can only consist of the active ingredient and cross-linked polyvinylpyrrolidone . Furthermore, one or more water soluble polymers can be added to improve the physical property of the present dispersion. The water soluble polymers are the common soluble polymers used in the art which may be selected from but not limited to the group comprising:
Alkylcellulose, such as methylcellulose;
Hydroxyalkylcellulose, such as hydroxymethylcellulose, hydroxyethylcellulose, hydroxypropylcellulose, hydroxybutylcellulose;
Hydroxyalkylalkylcellulose, such as hydroxyethylmethylcellulose, hydroxypropylmethylcellulose;
Carboxyalkylcellulose, such as carmellose;
Alkali metal salts of carboxyalkylcellulose, such as carboxymethylcellulose sodium; Carboxyalkylalkylcellulose, such as carboxymethylethylcellulose;
Carboxyalkylcellulose esters;
Pectin, such as carboxymethyl amylopectin sodium;
Chitin derivatives, such as chitosan;
Polysaccharide, such as alginic acid and the alkali metal salts or ammonium salts thereof, carrageenans, galactomannans, tragacanth gum, agar, acacia, guar gum, and xanthum gum;
Polymethacrylic acid and the salts thereof;
Polymethacrylic acid and the salts thereof, methacrylate copolymer, aminoalkyl methacrylate copolymer;
Polyvinyl acetal and diethylamino acetate;
Sugar surfactant; Polyvinyl alcohol;
Polyvinylpyrrolidone and polyvinylpyrrolidone-vinyl acetate copolymer;
Polyalkylene oxide, such as polyethylene oxide and polypropylene oxide;
Or ethylene oxide - propylene oxide copolymers.

In the above water soluble polymers, alkylcellulose, hydroxyalkylcellulose, carboxyalkylalkylcellulose or the alkali metal salts thereof, polyvinylpyrrolidone are preferable, and hydroxypropylmethylcellulose, polyvinylpyrrolidone are more preferable. In a preferred embodiment, ratio of water soluble polymers and cross-linked polyvinylpyrrolidone by weight is in the range of 1:5∼20, more preferably 1:8∼15,most preferably 1:10.

It is showed that as the water soluble polymers can improve the property of the solid dispersion, so there is no limited to the content of the water soluble polymers. The person skilled in the art can choose the content according to the practical production. It is showed in the research that when the ratio of water soluble polymers and cross-linked polyvinylpyrrolidone reaches 1:5∼20, more preferably 1:8∼15, most preferably 1:10, the preparation shows a statistical improvement effect.

Moreover, the composition described in the examples can best prove the effect of the present invention. The preferred embodiment of the solid dispersion in the present invention is:

| Component | Weight ratio |
|---|---|
| Tolvaptan | 2 |
| Cross-linked polyvinylpyrrolidone | 1 |
| Water-soluble polymers | 0.1 |

Wherein the water soluble polymers are polyvinylpyrrolidone, hydroxypropylcellulose, hydroxyethylcellulose or methylcellulose, and polyvinylpyrrolidone or hydroxypropylcellulose is preferred.

When water soluble polymers are contained in the composition, the solid dispersion can only consist of the active ingredient, cross-linked polyvinylpyrrolidone and water soluble polymers without any other components.

In the preparation of the amorphous tolvaptan solid dispersion, cross-linked polyvinylpyrrolidone can be used singly or in combination with water soluble polymers as the carrier. Practical preparation includes the following steps:
(1) tolvaptan or salt thereof is dissolved in the organic solvent;
(2) When the solid dispersion does not contain water soluble polymers, cross-linked polyvinylpyrrolidone is dissolved or dispersed in the organic solvent;
   When the solid dispersion contains water soluble polymers, cross-linked polyvinylpyrrolidone and the water soluble polymers can be dissolved or dispersed in the organic solvent at the same time to obtain a solution or in two different organic solvents separately to obtain two solutions. The organic solvents may contain water according to the drug or the solid dispersion carrier;
(3) the solutions of the drug and the solid dispersion carrier are mixed, and the organic solvent is removed to obtain the solid dispersion composition.

To obtain the solid dispersion preparation, the organic solvent can be removed by the conventional methods in the art, such as evaporation, spray drying process or fluidized bed drying process. For the present invention, spray drying process is preferred.

Any organic solvent that can easily dissolve or disperse the drug and solid dispersion carrier above can be used. Examples of the organic solvent include lower alcohols such as methanol, ethanol, isopropanol; ketones such as acetone,butanone; halogenated hydrocarbons such as dichloromethane, dichloroethane, trichloromethane, carbon tetrachloride and the mixture thereof. At the same time, water may be added if necessary. Such as methanol, ethanol, isopropanol, acetone, butanone, dichloromethane, dichloroethane, trichloromethane, or carbon tetrachloride. Among them, the mixture of lower alcohol and halogenated hydrocarbons are particularly preferred in terms of solubility, distillation. The mixture of dichloromethane and methanol or ethanol is particularly preferred. In a preferred embodiment, the mixture of ethanol and dichloromethane is used to dissolve tolvaptan or the its salt, and the mixture of ethanol and dichloromethane is used to dissolve cross-linked polyvinylpyrrolidone and water soluble polymers.

The range of the particle diameters of the amorphous solid dispersion of the present invention is in the range of 0.01∼400µm, preferably 0.1∼300µm, more preferably 1∼200µm.

The tolvaptan solid dispersion of the present invention shows no endothermic peak in the DSC curve and no crystallization refraction peak in the X-ray powder diffraction pattern which proves that the tolvaptan included in the dispersion is present as stable amorphous form.

Moreover, the present invention provides a pharmaceutical composition comprising the solid dispersion of the present invention and the pharmaceutical acceptable carrier, excipient, or additive in the art for oral administration. The pharmaceutical composition can be prepared into conventional dosage forms, such as powders, granules, tablets, soft or hard capsules, pills or coated forms. For example, the solid dispersion in the form of powder or granule can be filled into the hard capsule with lubricants or other pharmaceutical additives, or can be pressed into tablets with the pharmaceutical additives then coated using conventional method to obtain coating forms.

The present preparation can be administered orally in single dose or separate dose.

In the present invention, one or more pharmaceutical excipients may be added optionally into the preparation of the solid dispersion composition administered orally to improve the flowability and other physical properties. The pharmaceutical excipient can be selected from the group consist of lactose, starch, sodium carboxymethyl starch, polyvinylpyrrolidone, cross-linked carboxymethylcellulose sodium, maltose dextrin, crystalline cellulose, calcium phosphate, the mixture of calcium bicarbonate and crystalline cellulose. Furthermore, lubricants such as stearic acid, magnesium stearate and talc may be added.

### BRIEF DESCRIPTION OF THE DRAWINGS

The objects and characteristics of the present invention can be obvious by the following figures. The figures shows that:

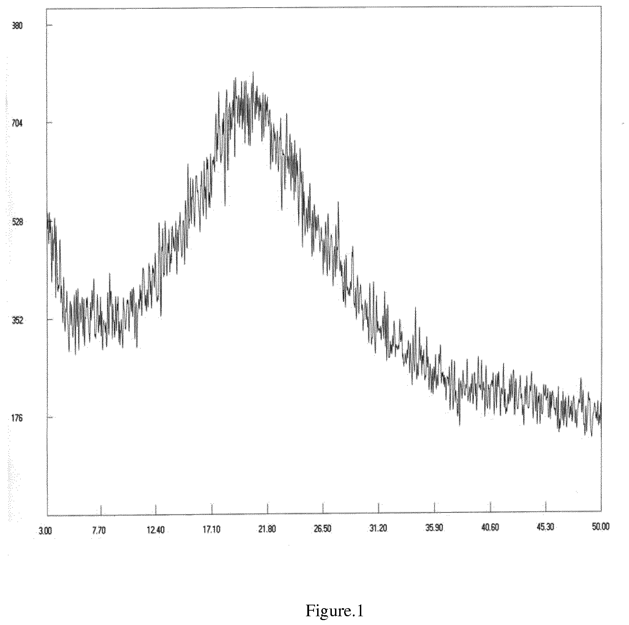

Figure. 1: the X-ray powder diffraction pattern of Example 1;

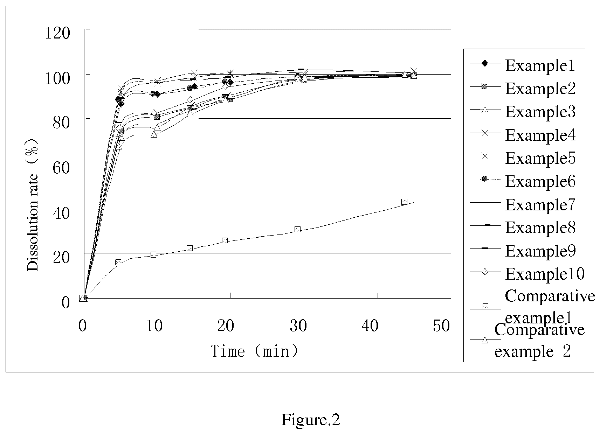

Figure.2: the *in vitro* release profile of Example1-10, Comparative example1-2;

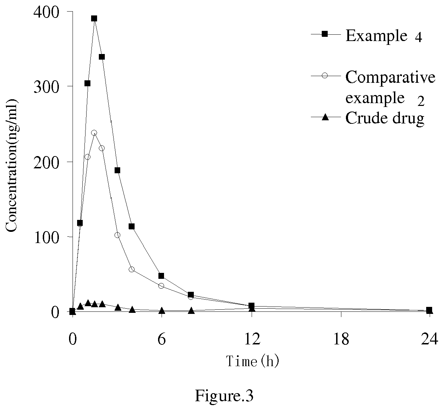

Figure.3: the *in vitro* bioavailability profile of raw material, Example 4, and Comparative example 2.

### DETAILED DESCRIPTION OF THE INVENTION

The present invention is more specifically described by following examples, however, the present invention is not limited to those examples in any way.

### Example 1

20g tolvaptan was added to the mixture of dichloromethane and ethanol, and stirred until the solution was clear. 10g cross-linked polyvinylpyrrolidone was added to ethanol and dispersed well. The solution of tolvaptan and the dispersion of cross-linked polyvinylpyrrolidone were mixed and spray-dried to give the tolvaptan solid dispersion which has a particle size distribution of 1µm∼400µm and an average particle diameter of 52µm.

### Example 2

20g tolvaptan was added to the mixture of dichloromethane and ethanol, and stirred until the solution was clear. 2g cross-linked polyvinylpyrrolidone was added to ethanol and dispersed well. The solution of tolvaptan and the dispersion of cross-linked polyvinylpyrrolidone were mixed and spray-dried to give the tolvaptan solid dispersion which has a particle size distribution of 1µm∼400µm and an average particle diameter of 52µm.

### Example 3

20g tolvaptan was added to the mixture of dichloromethane and ethanol, and stirred until the solution was clear. 200g cross-linked polyvinylpyrrolidone was added to ethanol and dispersed well. The solution of tolvaptan and the dispersion of cross-linked polyvinylpyrrolidone were mixed and spray-dried to give the tolvaptan solid dispersion which has a particle size distribution of 1 µm∼400µm and an average particle diameter of 52µm.

### Example 4

20g tolvaptan was added to the mixture of dichloromethane and ethanol and stirred until the solution was clear. 1g polyvinylpyrrolidone was added to the mixture of dichloromethane and ethanol and stirred until the solution was clear. 10g cross-linked polyvinylpyrrolidone was added to the mixture of dichloromethane and ethanol and dispersed well. The solution of tolvaptan and the dispersion of other ingredients were mixed and spray-dried to give the tolvaptan solid dispersion which has a particle size distribution of 1µm∼100µm and an average particle diameter of 25µm.

### Example 5

20g tolvaptan was added to the mixture of dichloromethane and ethanol and stirred until the solution was clear. 1g hydroxypropylmethyl cellulose was added to the mixture of dichloromethane and ethanol and stirred until the solution was clear. 10g cross-linked polyvinylpyrrolidone was added to the mixture of dichloromethane and ethanol and dispersed well. The solution of tolvaptan and the dispersion of other ingredients were mixed and spray-dried to give the tolvaptan solid dispersion which has a particle size distribution of 1µm∼100µm and an average particle diameter of 25µm.

### Example 6

20g tolvaptan was added to the mixture of dichloromethane and ethanol and stirred until the solution was clear. 1g hydroxyethyl cellulose was added to the mixture of dichloromethane and ethanol and stirred until the solution was clear. 10g cross-linked polyvinylpyrrolidone was added to the mixture of dichloromethane and ethanol and dispersed well. The solution of tolvaptan and the dispersion of other ingredients were mixed and spray-dried to give the tolvaptan solid dispersion which has a particle size distribution of 1µm∼100µm and an average particle diameter of 25µm.

### Example 7

20g tolvaptan was added to the mixture of dichloromethane and ethanol and stirred until the solution was clear. 1g carboxymethyl cellulose sodium was added to the mixture of dichloromethane and ethanol and stirred until the solution was clear. 10g cross-linked polyvinylpyrrolidone was added to the mixture of dichloromethane and ethanol and dispersed well. The solution of tolvaptan and the dispersion of other ingredients were mixed and spray-dried to give the tolvaptan solid dispersion which has a particle size distribution of 1µm∼100µm and an average particle diameter of 25µm.

### Example 8

20g tolvaptan was added to the mixture of dichloromethane and ethanol and stirred until the solution was clear. 1g methylcellulose was added to the mixture of dichloromethane and ethanol and stirred until the solution was clear. 10g cross-linked polyvinylpyrrolidone was added to the mixture of dichloromethane and ethanol and dispersed well. The solution of tolvaptan and the dispersion of other ingredients were mixed and spray-dried to give the tolvaptan solid dispersion which has a particle size distribution of 1µm∼100µm and an average particle diameter of 25µm.

### Example 9

20g tolvaptan was added to the mixture of dichloromethane and ethanol and stirred until the solution was clear. 2g sucrose ester was added to the mixture of dichloromethane and ethanol and stirred until the solution was clear. 10g cross-linked polyvinylpyrrolidone was added to the mixture of dichloromethane and ethanol and dispersed well. The solution of tolvaptan and the dispersion of other ingredients were mixed and spray-dried to give the tolvaptan solid dispersion which has a particle size distribution of 1µm∼100µm and an average particle diameter of 25µm.

### Example 10

20g tolvaptan was added to the mixture of dichloromethane and ethanol and stirred until the solution was clear. 0.5g polyvinyl alcohol was added to the mixture of dichloromethane and ethanol and stirred until the solution was clear. 10g cross-linked polyvinylpyrrolidone was added to the mixture of dichloromethane and ethanol and dispersed well. The solution of tolvaptan and the dispersion of other ingredients were mixed and spray-dried to give the tolvaptan solid dispersion which has a particle size distribution of 1µm∼100µm and an average particle diameter of 25µm.

### Comparative example 1

10g tolvaptan was added to the mixture of dichloromethane and ethanol and stirred until the solution was clear. The solution was spray-dried directly to give the tolvaptan amorphous powder which has a particle diameter distribution of 0.01 µm∼200µm and an average particle diameter of 28µm.

### Comparative example 2

20g tolvaptan was added to the mixture of dichloromethane and ethanol and stirred until the solution was clear. Then 10g hydroxypropylcellulose was added to the solution and dispersed well. The mixture was spry-dried to give the tolvaptan solid dispersion which has a particle diameter distribution of 1 µm∼400µm and an average particle diameter of 56µm.

### Dissolution test

Q.S. (the amount is equal to 100mg of tolvaptan) of the powder prepared in Example1-10, Comparative examples and the control (crystal material with a particle diameter distribution of 1∼400µm and an average diameter of 83µm) were added to the dissolution tester. 0.2% w/v sodium dodecylsulfate aqueous solution was used as solvent. The dissolution test was performed using paddle blade method with the rotation speed: 100rpm. The sample was taken at 5min, 10min, 15min, 20min, 30min, 45min to determine the absorbance and calculate the dissolution rate.

The determination of dissolution rate (%) is as following: dissolve certain amount of tolvaptan in methanol; 5ml of the solution was transferred to a 100-ml volumetric flask and then diluted with 0.2% (w/v) sodium dodecylsulfate aqueous solution to give a 20µg/ml standard solution; the absorbance of the standard solution and the sample solution was determined at wavelength of 269nm and 330nm. The dissolution rate can be obtained by the ratio of the difference of the absorbance of standard solution and the control and the difference of the absorbance of the sample solution and control. The results are shown in the following Table 1.

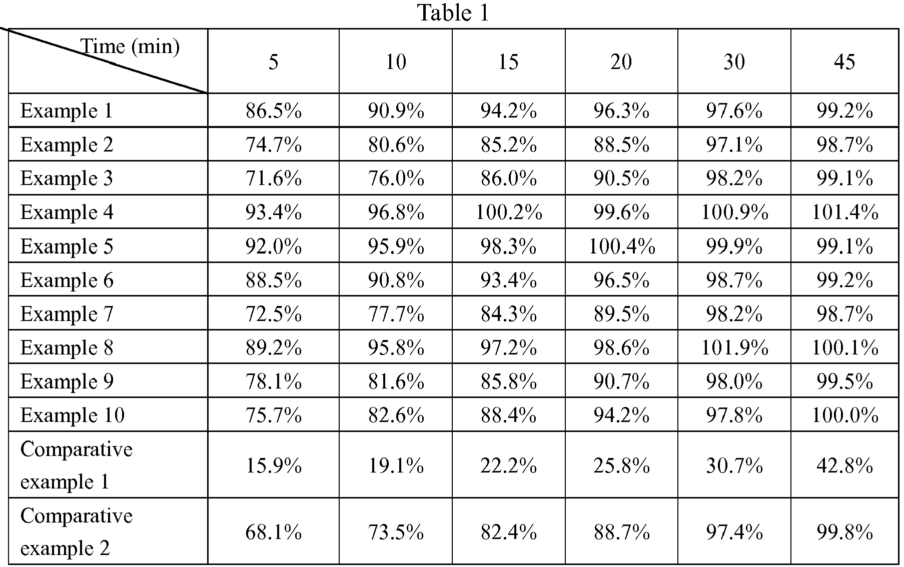

The results show that:
(i) The release characteristic is improved significantly by using cross-linked polyvinylpyrrolidone as the carrier of the tolvaptan solid dispersion, compared to solid dispersion using hydroxypropylcellulose as the carrier in the art, the effect improved significantly. The ratio of the drug and the cross-linked polyvinylpyrrolidone by weight can be selected in a large range, the ratio in the range of 1:0.1-10 is preferable and the ratio in the range of 1:0.5 is thus excellent.
(ii) Other water-soluble polymers can be used as carrier in combination with cross-linked polyvinylpyrrolidone on the basis of using cross-linked polyvinylpyrrolidone as the carrier of tolvaptan solid dispersion. After comparison, it is found that as the water-soluble polymers can further improve the properties of the solid dispersion, so there is no limitation to the amount of the water-soluble polymers.
When the ratio of the water-soluble polymer and cross-linked polyvinylpyrrolidone reaches 1:5-20, it shows a statistical improvement effect, more preferably 1:10.

Among all the water-soluble polymers, hydroxyethyl cellulose and methylcellulose are preferable, and hydroxypropylcellulose and polyvinylpyrrolidone are most preferable.

### Example 11

| | |
|---|---|
| the solid dispersion prepared in Example 1 | 18g |
| Lactose | 24g |
| maize starch | 6g |
| microcrystalline cellulose | 6g |
| 5% hydroxypropylcellulose solution | q.s. |
| cross-linked polyvinylpyrrolidone | 3g |
| magnesium stearate | 0.3g |

The solid dispersion was sieved through a 40-mesh sieve, the excipients were sieved through a 80-mesh sieve. The solid dispersion and the excipients were mixed well. 5% hydroxypropyl cellulose solution was used to granulate, and then dried by aeration-drying at 50°C. The dry-granules were sieved through a 20-mesh sieve. Cross-linked polyvinylpyrrolidone and magnesium stearate were added and mixed well. the mixture was filled into the capsules.

### Example 12

| | |
|---|---|
| the solid dispersion prepared in Example 3 | 58g |
| lactose | 15g |
| microcrystalline cellulose | 5g |
| 5% hydroxypropylcellulose solution | q.s. |
| Cross-linked carmellose | 3g |
| magnesium stearate | 0.3g |
| opadry | 1.8g |

The solid dispersion was sieved through a 40-mesh sieve, the excipients were sieved through a 80-mesh sieve. The solid dispersion and the excipients were mixed well. 5% hydroxypropyl cellulose solution was used to granulate, and then dried by aeration-drying at 50 °C. The dry-granules were sieved through a 20-mesh sieve. Cross-linked carmellose and magnesium stearate were added and mixed well. The mixture was made into tablets and coated.

### Example 13

| | |
|---|---|
| the solid dispersion prepared in Example 2 | 95g |
| lactose | 10g |
| pregelatinized starch | 2g |
| microcrystalline cellulose | 2g |
| 5% polyvinylpyrrolidone solution | q.s. |
| low substituted hydroxypropylcellulose | 5g |
| magnesium stearate | 0.3g |

The solid dispersion was sieved through a 40-mesh sieve, the excipients were sieved through a 80-mesh sieve. The solid dispersion and the excipients were mixed well. 5% polyvinylpyrrolidone solution was used to granulate, and then dried by aeration-drying at 50 °C. The dry-granules were sieved through a 20-mesh sieve. Low substituted hydroxypropylcellulose and magnesium stearate were added and mixed well. The mixture was made into tablets.

## Claims

1. A pharmaceutical solid dispersion comprising amorphous tolvaptan or salts thereof as active ingredient and cross-linked polyvinylpyrrolidone, wherein the ratio of the active ingredient tolvaptan and the cross-linked polyvinylpyrrolidone by weight is 1 :0.1-10.

2. The pharmaceutical solid dispersion according to claim 1, wherein the ratio of the active ingredient tolvaptan and the cross-linked polyvinylpyrrolidone by weight, is 2:1.

3. The pharmaceutical solid dispersion according to claim 1, wherein the solid dispersion is consisting of the active ingredient and the cross-linked polyvinylpyrrolidone.

4. The pharmaceutical solid dispersion according to any one of claims 1 to 3, wherein the solid dispersion further comprises water soluble polymers.

5. The pharmaceutical solid dispersion according to claim 4, wherein the solid dispersion is consisting of the active ingredient, the cross-linked polyvinylpyrrolidone and the water soluble polymers.

6. The pharmaceutical solid dispersion according to claim 4, wherein the water soluble polymer is one or more selected from the group consisting of alkylcellulose, hydroxyalkylcellulose or alkali metal salts thereof, hydroxyalkylalkylcellulose, carboxyalkylcellulose, carboxyalkylalkyl cellulose, carboxyalkylcellulose ester, starch, pectin, chitin, polysaccharide, polyvinylpyrrolidone.

7. The pharmaceutical solid dispersion according to claim 6, wherein the water soluble polymer is one or more selected from the group consisting of alkylcellulose, hydroxyalkylcellulose, carboxyalkylcellulose or alkali metal salt thereof, polyvinylpyrrolidone.

8. The pharmaceutical solid dispersion according to claim 6, wherein the water soluble polymer is one or more selected from the group consisting of hydroxypropylcellulose polyvinylpyrrolidone.

9. The pharmaceutical solid dispersion according to any one of claims 5 to 8, wherein the ratio of the water soluble polymer and the cross-linked polyvinylpyrrolidone by weight is 1:5-20.

10. The pharmaceutical solid dispersion according to any one of claim 9, wherein the ratio of the water soluble polymer and the cross-linked polyvinylpyrrolidone by weight is 1:8-15.

11. The pharmaceutical solid dispersion according to claim 9, wherein the ratio of the water soluble polymer and the cross-linked polyvinylpyrrolidone by weight is 1:10.

12. The pharmaceutical solid dispersion according to claim 1, comprising tolvaptan, cross-linked polyvinylpyrrolidone and water soluble polymers at a ratio of 2:1:0.1 by weight, wherein the water soluble polymer is polyvinylpyrrolidone, hydroxypropylcellulose, hydroxyethylcellulose or methylcellulose.

13. The pharmaceutical solid dispersion according to claim 12, consisting of tolvaptan, cross-linked polyvinylpyrrolidone, and water soluble polymers.

14. A preparation method of the pharmaceutical solid dispersion according to any one of claims 1 to 13, comprising:
i) tolvaptan or salts thereof is dissolved in a organic solvent;
ii) cross-linked polyvinylpyrrolidone is dissolved or dispersed in a organic solvent, or cross-linked polyvinylpyrrolidone and water soluble polymers are dissolved or dispersed in a organic solvent, or cross-linked polyvinylpyrrolidone and water soluble polymers are dissolved or dispersed in a organic solvent separately, the organic solvents may contain water according to the necessity of dissolving and dispersing.
iii) the aforementioned solutions are mixed, the organic solvent is removed to give the solid dispersion mixture.

15. The preparation method according to claim 14, wherein the way to remove the organic solvent is selected from evaporation, spray drying method, fluidized bed drying method.

16. The preparation method according to claim 14, wherein the organic solvent used in Step i) or Step ii) is selected from the group comprising methanol, ethanol, isopropanol, acetone, butanone, dichloromethane, dichloroethane, trichloromethane, and carbon tetrachloride.

17. The method according to claim 15, wherein the organic solvent used in Step i) is the mixture of ethanol and dichloromethane; and/or the organic solvent used in Step ii) is the mixture of ethanol and dichloromethane or ethanol.

18. A pharmaceutical composition comprising the solid dispersion according to any one of claims 1 to 13.

## Patentansprüche

1. Pharmazeutische Feststoffdispersion, die amorphes Tolvaptan oder Salze davon als Wirkstoff und vernetztes Polyvinylpyrrolidon enthält, wobei das Gewichtsverhältnis des Wirkstoffs Tolvaptan und des vernetzten Polyvinylpyrrolidons 1:0,1-10 beträgt.

2. Pharmazeutische Feststoffdispersion nach Anspruch 1, wobei das Gewichtsverhältnis des Wirkstoffs Tolvaptan und des vernetzten Polyvinylpyrrolidons 2:1 beträgt.

3. Pharmazeutische Feststoffdispersion nach Anspruch 1, wobei die Feststoffdispersion aus dem Wirkstoff und dem vernetzten Polyvinylpyrrolidon besteht.

4. Pharmazeutische Feststoffdispersion nach einem der Ansprüche 1 bis 3, wobei die Feststoffdispersion ferner wasserlösliche Polymere enthält.

5. Pharmazeutische Feststoffdispersion nach Anspruch 4, wobei die Feststoffdispersion aus dem Wirkstoff, dem vernetzten Polyvinylpyrrolidon und den wasserlöslichen Polymeren besteht.

6. Pharmazeutische Feststoffdispersion nach Anspruch 4, wobei das wasserlösliche Polymer ein oder mehrere aus der Gruppe bestehend aus Alkylcellulose, Hydroxyalkylcellulose oder Alkalimetallsalze davon, Hydroxyalkylalkylcellulose, Carboxyalkylcellulose, Carboxyalkylalkylcellulose, Carboxyalkylcelluloseester, Stärke, Pektin, Chitin, Polysaccharid, Polyvinylpyrrolidon ausgewählt ist.

7. Pharmazeutische Feststoffdispersion nach Anspruch 6, wobei das wasserlösliche Polymer ein oder mehrere aus der Gruppe bestehend aus Alkylcellulose, Hydroxyalkylcellulose, Carboxyalkylcellulose oder Alkalimetallsalze davon, Polyvinylpyrrolidon ausgewählt ist.

8. Pharmazeutische Feststoffdispersion nach Anspruch 6, wobei das wasserlösliche Polymer ein oder mehrere aus der Gruppe bestehend aus Hydroxypropylcellulose und Polyvinylpyrrolidon ausgewählt ist.

9. Pharmazeutische Feststoffdispersion nach einem der Ansprüche 5 bis 8, wobei das Gewichtsverhältnis des wasserlöslichen Polymers und des vernetzten Polyvinylpyrrolidons 1:5-20 beträgt.

10. Pharmazeutische Feststoffdispersion nach Anspruch 9, wobei das Gewichtsverhältnis des wasserlöslichen Polymers und des vernetzten Polyvinylpyrrolidons 1:8-15 beträgt.

11. Pharmazeutische Feststoffdispersion nach Anspruch 9, wobei das Gewichtsverhältnis des wasserlöslichen Polymers und des vernetzten Polyvinylpyrrolidons 1:10 beträgt.

12. Pharmazeutische Feststoffdispersion nach Anspruch 1, die Tolvaptan, vernetztes Polyvinylpyrrolidon und wasserlösliche Polymere im Gewichtsverhältnis 2:1:0,1 enthält, wobei das wasserlösliche Polymer Polyvinylpyrrolidon, Hydroxypropylcellulose, Hydroxyethylcellulose oder Methylcellulose ist.

13. Pharmazeutische Feststoffdispersion nach Anspruch 12, bestehend aus Tolvaptan, vernetztem Polyvinylpyrrolidon und wasserlöslichen Polymeren.

14. Zubereitungsverfahren der pharmazeutischen Feststoffdispersion nach einem der Ansprüche 1 bis 13, das folgendes umfasst:
i) Tolvaptan oder Salze davon in einem organischen Lösungsmittel gelöst sind;
ii) vernetztes Polyvinylpyrrolidon in einem organischen Lösungsmittel gelöst oder dispergiert ist, oder vernetztes Polyvinylpyrrolidon und wasserlösliche Polymere in einem organischen Lösungsmittel gelöst oder dispergiert sind, oder vernetztes Polyvinylpyrrolidon und wasserlösliche Polymere in einem organischen Lösungsmittel getrennt gelöst oder dispergiert sind, wobei die organischen Lösungsmittel je nach Notwendigkeit des Lösens und Dispergierens Wasser enthalten können;
iii) die oben genannten Lösungen gemischt werden, das organische Lösungsmittel entfernt wird, um zur festen Dispersionsmischung zu gelangen.

15. Zubereitungsverfahren nach Anspruch 14, wobei der Weg zur Entfernung des organischen Lösungsmittels unter Verdunstung, Sprühtrocknungsverfahren, Wirbelschichttrocknungsverfahren ausgewählt ist.

16. Zubereitungsverfahren nach Anspruch 14, wobei das in Schritt i) oder Schritt ii) verwendete organische Lösungsmittel aus der Gruppe umfassend Methanol, Ethanol, Isopropanol, Aceton, Butanon, Dichlormethan, Dichlorethan, Trichlormethan und Kohlenstofftetrachlorid ausgewählt ist.

17. Zubereitungsverfahren nach Anspruch 15, wobei das in Schritt i) verwendete organische Lösungsmittel die Mischung aus Ethanol und Dichlormethan ist; und/oder das in Schritt ii) verwendete organische Lösungsmittel die Mischung aus Ethanol und Dichlormethan oder Ethanol ist.

18. Pharmazeutische Zusammensetzung, die die Feststoffdispersion nach einem der Ansprüche 1 bis 13 enthält.

## Revendications

1. Dispersion pharmaceutique solide comprenant du tolvaptan amorphe ou des sels de celui-ci à titre de principe actif et une polyvinylpyrrolidone réticulée, dans laquelle le rapport du principe actif tolvaptan à la polyvinylpyrrolidone réticulée en poids est de 1/0,1-10.

2. Dispersion pharmaceutique solide selon la revendication 1, dans laquelle le rapport du principe actif tolvaptan à la polyvinylpyrrolidone réticulée en poids est de 2/1.

3. Dispersion pharmaceutique solide selon la revendication 1, dans laquelle la dispersion solide est constituée du principe actif et de la polyvinylpyrrolidone réticulée.

4. Dispersion pharmaceutique solide selon l'une quelconque des revendications 1 à 3, dans laquelle la dispersion solide comprend en outre des polymères hydrosolubles.

5. Dispersion pharmaceutique solide selon la revendication 4, dans laquelle la dispersion solide est constituée du principe actif, de la polyvinylpyrrolidone réticulée et de polymères hydrosolubles.

6. Dispersion pharmaceutique solide selon la revendication 4, dans laquelle le polymère hydrosoluble est un ou plusieurs polymères choisis dans le groupe constitué par une alkylcellulose, une hydroxyalkylcellulose ou ses sels de métal alcalin, une hydroxyalkylalkylcellulose, une carboxyalkylcellulose, une carboxyalkylalkylcellulose, un ester de carboxyalkyl-cellulose, l'amidon, la pectine, la chitine, un polysaccharide, la polyvinylpyrrolidone.

7. Dispersion pharmaceutique solide selon la revendication 6, dans laquelle le polymère hydrosoluble est un ou plusieurs polymères choisis dans le groupe constitué par une alkylcellulose, une hydroxyalkylcellulose, une carboxyalkyl-cellulose ou un sel de métal alcalin de celle-ci, la polyvinylpyrrolidone.

8. Dispersion pharmaceutique solide selon la revendication 6, dans laquelle le polymère hydrosoluble est un ou plusieurs polymères choisis dans le groupe constitué par l'hydroxypropylcellulose et la polyvinylpyrrolidone.

9. Dispersion pharmaceutique solide selon l'une quelconque des revendications 5 à 8, dans laquelle le rapport du polymère hydrosoluble à la polyvinylpyrrolidone réticulée en poids est de 1/5-20.

10. Dispersion pharmaceutique solide selon la revendication 9, dans laquelle le rapport du polymère hydrosoluble à la polyvinylpyrrolidone réticulée en poids est de 1/8-15.

11. Dispersion pharmaceutique solide selon la revendication 9, dans laquelle le rapport du polymère hydrosoluble à la polyvinylpyrrolidone réticulée en poids est de 1/10.

12. Dispersion pharmaceutique solide selon la revendication 1, comprenant du tolvaptan, une polyvinylpyrrolidone réticulée et des polymères hydrosolubles dans un rapport de 2/1/0,1 en poids, dans laquelle le polymère hydrosoluble est la polyvinylpyrrolidone, l'hydroxypropylcellulose, l'hydroxyéthyl-cellulose ou la méthylcellulose.

13. Dispersion pharmaceutique solide selon la revendication 12, constituée de tolvaptan, polyvinylpyrrolidone réticulée et polymères hydrosolubles.

14. Procédé de préparation de la dispersion pharmaceutique solide selon l'une quelconque des revendications 1 à 13, comprenant :
i) la dissolution du tolvaptan ou de ses sels dans un solvant organique ;
ii) la dissolution ou la dispersion de la polyvinylpyrrolidone réticulée dans un solvant organique, ou la dissolution ou la dispersion de la polyvinylpyrrolidone réticulée et des polymères hydrosolubles dans un solvant organique, ou la dissolution ou la dispersion séparée de la polyvinylpyrrolidone réticulée et des polymères hydrosolubles dans un solvant organique, où les solvants organiques peuvent contenir de l'eau selon les besoins pour la dissolution et dispersion ;
iii) le mélange des solutions précitées et l'élimination du solvant pour obtenir un mélange de type dispersion solide.

15. Procédé de préparation selon la revendication 14, dans lequel le moyen d'élimination du solvant organique est choisi parmi l'évaporation, un procédé de lyophilisation, un procédé de séchage en lit fluidisé.

16. Procédé de préparation selon la revendication 14, dans lequel le solvant organique utilisé à l'Etape i) ou l'Etape ii) est choisi dans le groupe comprenant le méthanol, l'éthanol, l'isopropanol, l'acétone, la butanone, le dichlorométhane, le dichloroéthane, le trichlorométhane, et le tétrachlorure de carbone.

17. Procédé de préparation selon la revendication 15, dans lequel le solvant organique utilisé à l'Etape i) est le mélange éthanol et dichlorométhane ; et/ou le solvant organique utilisé à l'Etape ii) est le mélange éthanol et dichlorométhane ou l'éthanol.

18. Composition pharmaceutique comprenant la dispersion solide selon l'une quelconque des revendications 1 à 13.
